# EUROPÄISCHE PATENTSCHRIFT

(11) **EP 1 093 755 B1**
(45) Veröffentlichungstag und Bekanntmachung des Hinweises auf die Patenterteilung: **19.03.2008**
(21) Anmeldenummer: 00121537.5
(22) Anmeldetag: 30.09.2000
(51) Int. Cl.: A61B 5/103, A61G 7/057, A61B 5/11

(54) **Vorrichtung zum Messen von Werten einer liegenden Person**
Device for measuring parameters of a lying person
Dispositif de mesure de paramètres d'une personne couchée

(30) Priorität: 19.10.1999 DE 19950291; 18.01.2000 DE 10001698
(43) Veröffentlichungstag der Anmeldung: 25.04.2001
(73) Patentinhaber: Thomas Hilfen HILBEG GmbH & Co. Kommanditgesellschaft, 27432 Bremervörde (DE)
(72) Erfinder: Jansen, Klaus, Dr., 21614 Buxtehude (DE); Vent, Michael, 21641 Apensen (DE)
(74) Vertreter: Möller, Friedrich

(56) Entgegenhaltungen:
- WO-A-98/34577
- DE-A- 4 240 782
- DE-U- 9 100 370
- FR-A- 2 720 622
- US-A- 2 644 332
- US-A- 4 175 263

## Beschreibung

Die Erfindung betrifft eine Vorrichtung zum Messen von Werten einer liegenden Person, insbesondere deren Bewegung, Bewegungsrichtung und/oder Vitalparameter, mit Sensoren. Ferner

US-A-4 175 263 offenbart eine Vorrichtung zum Messen von Werten einer auf einer Matratze liegenden Person, insbesondere deren Bewegung und Bewegungsrichtung, mit zwei an der Matratze angeordneten und jeweils einen großflächigen Messbereich abdeckenden Sensoren, die zum Messen im Wesentlichen statischer Werte der Person ausgebildet sind.

Es ist bekannt, Druckwerte von Körperpartien einer auf einer Matratze liegenden Person zu messen. Derartige Messungen dienen in erster Linie der Dekubitusprophylaxe. Eine aus DE 42 40 782 A1 bekannte Vorrichtung weist hierzu eine Vielzahl von Sensoren, nämlich 33 Reihen mit jeweils 23 Sensoren und damit insgesamt mehr als 600 Sensoren, auf, welche die Druckbelastungen messen und erhaltene Messwerte an eine Auswerteeinheit übertragen.

Diese bekannte Vorrichtung hat den Nachteil, dass sie aufgrund der großen Anzahl von Sensoren störanfällig ist. Ferner muss jeder einzelne Sensor kalibriert werden, damit die Messergebnisse der einzelnen Sensoren miteinander vergleichbar sind. Dies erfordert einen sehr hohen Aufwand, insbesondere da diese Kalibrierung in bestimmten Wartungsintervallen wiederholt werden muss.

Der Erfindung liegt daher das technische Problem zugrunde, das Messen von Werten einer liegenden Person zu verbessern.

Gelöst wird dieses Problems durch eine Vorrichtung gemäß Anspruch 1.

Die Sensoren sind erfindungsgemäß in dem Bereich der Vorrichtung angeordnet, in dem sich der Brustkorb einer auf der Matratze liegenden Person befindet - dem sogenannten Thoraxbereich - und/oder in dem Bereich, in dem sich der Unterleib der Person befindet, dem sog. Sakralbereich. Hierdurch befinden sich die Sensoren im Bereich des Schwerpunktes einer auf der Matratze liegenden Person. Dies erlaubt auf vorteilhafte Weise, Bewegungen sowie deren Richtung der Person zu erfassen. Darüber hinaus kann aufgrund der Anordnung eines Sensors im Thoraxbereich auch der Herzschlag sowie die Atmung der Person auf vorteilhafte Weise erfasst werden.

Bevorzugt werden Drücke mit einem Sensorkissen gemessen und in einer Auswerteeinheit ausgewertet, indem Absolutdrücke der Sensorkissen und/oder Relativdrücke zwischen mehreren Sensorkissen miteinander verrechnet werden, um eine Bewegung, deren Richtung und/oder Vitalparameter der Person zu ermitteln.

Ein erfindungsgemäßer Drucksensor ist dadurch gekennzeichnet, dass der Drucksensor ein Sensorkissen aufweist, das mit einem gasförmigen oder flüssigen Medium gefüllt ist, wobei ein auf das Sensorkissen ausübbarer Druck über das Medium auf das Sensorelement übertragbar ist. Dabei ist unter dem Begriff "Drucksensor" eine (größere) Anordnung mit einem Kissen - dem Sensorkissen - und wenigstens einem (im Vergleich zu dieser Anordnung kleineren) Sensorelement zu verstehen.

Ein in oder an der erfindungsgemäßen Vorrichtung bzw. Matratze angebrachter erfindungsgemäßer Drucksensor bietet den Vorteil, dass er die Aufnahmefläche und damit den Messbereich eines Sensorelements um ein Vielfaches gegenüber der Aufnahmefläche des Sensorelements selbst vergrößert. Dazu ist das Druckelement mit einem Kissen verbunden, das einen von einer sich auf der Matratze befindenden Person ausgeübten Druck auf das Sensorelement übertragen kann. Insgesamt erreicht man durch das angeschlossene Sensorkissen eine Oberflächenvergrößerung des Sensorelements. Dadurch genügt es, eine Matratze mit nur wenigen, insbesondere zwei bis vier oder fünf Drucksensoren, auszustatten, um gleichwohl einen großen Bereich der Matratze messtechnisch abzudecken.

Bevorzugt ist das Kissen von einer flüssigkeits- und/oder luftdichten Hülle umschlossen und mit einem gasförmigen oder flüssigen Medium gefüllt, das den Druck auf das Sensorelement weiterleitet. Bei diesem Medium kann es sich insbesondere um Luft, Wasser oder auch eine gelartige Substanz handeln.

Bevorzugt ist das Sensorkissen auch mit einem elastischen Material, beispielsweise Schaumstoff, gefüllt. Dies ist insbesondere vorteilhaft bei einem luftgefüllten Sensorkissen. Aufgrund der Füllung mit dem elastischen Material erhält nämlich das Sensorkissen immer wieder seine optimale Form. Dies gilt auch dann, wenn die an sich luftdichte Hülle beispielsweise durch geringfügige Beschädigung luftdurchlässig geworden ist und ein ausschließlich mit Luft gefülltes Kissen zusammenfallen würde. Eine Druckmessung mit einem derartigen Sensorkissen kann dann selbst bei geringen Beschädigungen der Kissenhülle durchgeführt werden.

Besonders bevorzugt ist das Sensorelement nicht direkt am Sensorkissen angebracht, sondern über einen Schlauch bzw. eine Schlauchkapillare mit den Sensorkissen verbunden. Dies hat u.a. den Vorteil, dass das empfindliche Sensorelement an einem mechanisch nicht oder nur wenig beanspruchten Bereich (der Matratze) untergebracht werden kann.

Aufgrund der vorteilhaften Auswertung der Absolutdrücke der Sensorkissen und/oder der Relativdrücke zwischen mehreren Sensorkissen kann man diverse Daten der sich auf der Matratze befindenden Person erfassen. Insbesondere aufgrund einer kombinierten Absolutdruckmessung und einer Messung der relativen Druckänderungen und ihres zeitlichen Verlaufs können Bewegungen einer sich auf der Matratze befindenden Person erkannt werden. Insbesondere erlaubt die Erfindung festzustellen, ob tatsächlich eine wesentliche Lageänderung bei einer Bewegung stattgefunden hat, bspw. von einer Seiten- in eine Rückenlage oder umgekehrt, oder ob es sich nur z.B. um eine Beinbewegung oder eine externe Manipulation des Bettes bzw. der Matratze ohne wesentliche Lageänderung des Körpers der Person gehandelt hat. Speziell diese Erkennung ist von Bedeutung, wenn eine Matratze im Bereich der Dekubitusprophylaxe eingesetzt werden soll.

Weitere Einzelheiten der Erfindung werden nachfolgend anhand der in der Zeichnung dargestellten Ausführungsbeispiele näher erläutert. Es zeigt:
- Fig. 1: den prinzipiellen Aufbau einer Matratze in Seitenansicht;
- Fig. 2: einen Drucksensor mit Sensorkissen und Sensorelement gemäß einem ersten Ausführungsbeispiel;
- Fig. 3: einen Drucksensor mit Sensorkissen und Sensorelement gemäß einer zweiten Ausführungsbeispiel;
- Fig. 4: die prinzipielle Anordnung von zwei Sensorkissen an einer Matratze in einer Ansicht von oben gemäß einem weiteren Ausführungsbeispiel;
- Fig. 5: die prinzipielle Anordnung von zwei Sensorkissen an einer Matratze in einer Ansicht von oben gemäß einem weiteren Ausführungsbeispiel;
- Fig. 6: die prinzipielle Anordnung von zwei Sensorkissen an einer Matratze in einer Ansicht von oben gemäß einem weiteren Ausführungsbeispiel;
- Fig. 7: die prinzipielle Anordnung von zwei Sensorkissen an einer Matratze in einer Ansicht von oben gemäß einem weiteren Ausführungsbeispiel;
- Fig. 8: die prinzipielle Anordnung von drei Sensorkissen an einer Matratze in einer Ansicht von oben gemäß einem weiteren Ausführungsbeispiel;
- Fig. 9: die prinzipielle Anordnung von drei Sensorkissen an einer Matratze in einer Ansicht von oben gemäß einem weiteren Ausführungsbeispiel;
- Fig. 10: die prinzipielle Anordnung von vier Sensorkissen an einer Matratze in einer Ansicht von oben gemäß einem weiteren Ausführungsbeispiel.

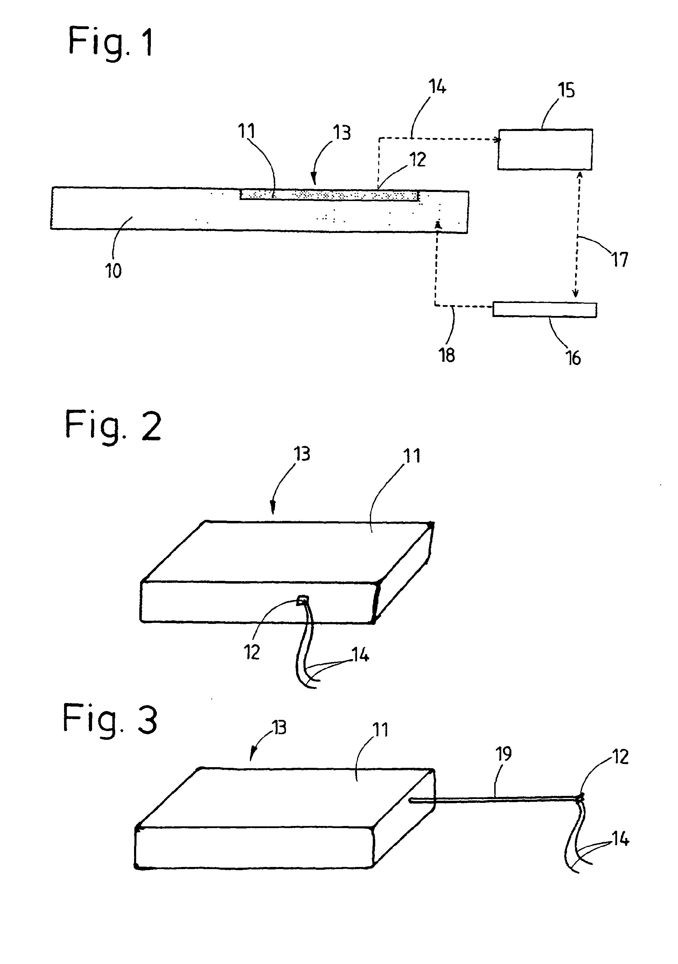

Fig. 1 zeigt eine Matratze 10 mit einem auf der Oberseite der Matratze eingelassenen Sensorkissen 11. Das Sensorkissen 11 weist eine derartige Gestalt auf, um in eine entsprechend ausgebildete Ausnehmung der Matratze 10 hinzupassen. Dabei sind die Gestalt und die Ausnehmung derart ausgebildet, dass die Oberfläche der Matratze auch im Bereich des Sensorkissens 11 keine Höhenunterschiede aufweist, d.h. dass die Oberfläche in einer Ebene liegt.

Das Sensorkissen 11 deckt einen großen Bereich der Oberseite der Matratze 10 ab. Die Abdeckung beträgt - bezogen auf die Fläche der Oberseite der Matratze - etwa 3% bis 50%, vorzugsweise 5% bis 25%, je Sensorkissen 11.

Ein Sensorelement bzw. Druckaufnehmer 12 ist am Sensorkissen 11 angebracht. Sensorelement bzw. Druckaufnehmer 12 und Sensorkissen 11 bilden einen sogenannten Drucksensor 13. Der Druckaufnehmer 12 ist über eine Messleitung 14 mit einer Auswerteeinheit 15 verbunden. Die Auswerteeinheit 15 führt die Messdatenaufnahme sowie deren Analyse durch. Die Auswerteeinheit 15 löst ferner geeignete Folgeoperationen aus. Diese Folgeoperationen können insbesondere in der Aktivierung einer Steuerungseinheit 16 bestehen, die über eine Datenleitung 17 bidirektional mit der Auswerteeinheit 15 verbunden ist. Über diese Datenleitung 17 werden Informationen bzw. Daten zwischen der Auswerteeinheit 15 und der Steuerungseinheit 16 ausgetauscht.

Die Steuerungseinheit 16 kann in Reaktion auf eine über die Datenleitung 17 erfolgte Eingabe der Auswerteeinheit 15 einen Matratzenkern in Bewegung versetzen. Dazu ist eine Steuerungsleitung 18 von der Steuerungseinheit 17 zur Matratze 10 vorgesehen. Die Steuerungsleitung 18 kann bspw. eine elektrische Leitung zur Übertragung elektrischer Energie sein, die zum elektrischen Antrieb elektrischer Bewegungsorgane, insbesondere elektrische Stellmotoren, innerhalb oder an der Matratze angeordnet sind. Die Steuerungsleitung kann jedoch auch eine hydraulische oder pneumatische Leitung sein, wobei die Steuerungseinheit 16 eine Flüssigkeit oder ein Gas, bspw. Luft, in die Matratze 10 pumpen kann.

Aufgrund dieser Bewegungen der Matratze 10 bzw. des Matratzenkerns kann ein bettlägeriger Partient bewegt und stimuliert werden. Hierdurch kann man einem Wundliegen vorbeugen. Insbesondere kann durch die Steuereinheit 16 und die an die Steuerungsleitung 18 angeschlossenen Organe die Position größten Auflagedrucks der auf der Matratze 10 liegenden Person verändert werden. Ferner können die Druckbelastungszonen einer liegenden Person durch eine Höhenverstellung der Organe verändert werden. Schließlich können auch die mit Luft oder Flüssigkeit gefüllten Sensorkissen 11 in ihrer Härte in Abhängigkeit von Steuerungsvorgaben der Steuerungseinheit 18 verändert werden, um für eine Druckentlastung der Person zu sorgen.

Fig. 2 zeigt ein Sensorkissen 11 in vergrößerter Darstellung in einer perspektivischen Ansicht. Auch das Sensorelement bzw. der Druckaufnehmer 12 ist vergrößert dargestellt. Er befindet sich bei diesem Ausführungsbeispiel in der Umhüllung des Sensorkissens 11 und ist über die Messleitung 14 mit der Auswerteeinheit 15 verbunden.

Das Sensorkissen 11 weist vorzugsweise quaderförmige Gestalt auf. Es kann jedoch auch andere Gestalten aufweisen oder aber auch ohne eine bevorzugte Form ausgestaltet sein. Beispielsweise ist es auch möglich, die Umhüllung des Sensorkissens 11 aus einem Material unspezifischer Form zu gestalten. Eine spezifische Form kann einem derartigen Kissen dann durch eine in der Form bestimmte Schaumstofffüllung gegeben werden. Auf diese Weise hat man die Möglichkeit durch einfachste Mittel ein Sensorkissen 11 an jede beliebig geartete Ausnehmung innerhalb einer Matratze 10 anzuordnen. Eine derartige Ausführung des Sensorkissens 11 ist auch dort vorteilhaft, wo das Sensorkissen 11 bspw. nachträglich im Inneren einer Matratze 10 untergebracht werden soll. Hier kann der Matratzenkern bzw. die Matratzenfüllung in beliebiger Weise entfernt werden, wobei anschließend das Kissen 11 in diese Ausnehmung genau einpassbar ist.

Fig. 3 zeigt eine weitere Variante des Sensorkissens 11 mit einem Schlauch bzw. eine Schlauchkapillare 19, die das Sensorelement 12 mit dem Sensorkissen 11 verbindet. Auf diese Weise kann das Sensorelement 12 und die Messleitungen 14 an einer von dem Kissen 11 entfernten Position untergebracht werden.

Mittels des beschriebenen Sensorkissens 11 lässt sich die Bewegung einer auf der Matratze 10 liegenden Person detektieren.

Dabei sind mehrere Sensorkissen 11 derart angeordnet, dass sie vom Oberkörper des Patienten teilweise oder vollständig bedeckt werden. Durch Bewegungen der Person werden in den Kissen 11 Druckänderungen verursacht. Der entstehende Druck wird über ein Medium - bspw. gasförmige Medien, wie Luft, oder flüssige Medien, wie Wasser, - an einen Druckaufnehmer übertragen. Derart entstandene Druckmuster werden mit den Druckaufnehmern aufgezeichnet und analysiert, so dass auf eine von der Person durchgeführte Bewegung geschlossen werden kann. Hierzu werden die aufgenommenen Messdaten in der Auswerteeinheit miteinander verrechnet. Zur Bewegungsdetektion sind dabei insbesondere zwei, drei, vier oder mehr Sensorkissen 11 vorgesehen. Dabei werden sowohl die Absolutdrücke der Sensorkissen als auch Relativdrücke zwischen mehreren Sensorkissen miteinander in rechnerische Beziehung gesetzt, so dass im Ergebnis ein bzw. mehrere Bewegungssignale erzeugt werden.

Es können jedoch auch weitere Signale aus der Physiologie der Person gewonnen werden. Insbesondere können mittels der Sensorkissen 11 auch Herztöne sowie Muskelbewegungen und die Atmung der Person erfasst werden. Hierfür ist bereits ein einzelnes Sensorkissen ausreichend, jedoch werden die Ergebnisse mit mehreren Kissen genauer. Insbesondere lassen sich mit mehreren Messungen verschiedener Sensorkissen etwaige Störungen herausfiltern bzw. rechnerisch unterdrücken.

Zusätzlich können auf der Oberseite der Matratze, insbesondere im Bereich der Sensorkissen 11 (nicht dargestellte) Temperatursensoren vorgesehen sein. Diese zusätzlichen Temperatursensoren erlauben ebenfalls eine Ermittlung der Lage der Person, da die Person Wärme an die Matratze abgibt. Durch eine Berücksichtigung von sowohl Temperaturwerten als auch Druckwerten erhöht man den Informationsgehalt der Messung und damit auch die Genauigkeit der gemessenen Messwerte. Die Temperaturmessung stellt insbesondere ein zusätzliches Plausibilitätskriterium zur Verfügung. Insbesondere der zeitliche Verlauf der Drücke und Temperaturmessungen erlaubt weitere Aussagen über das Verhalten der Person, sowie deren Vitalparameter, wie Atmung oder Herzschlag.

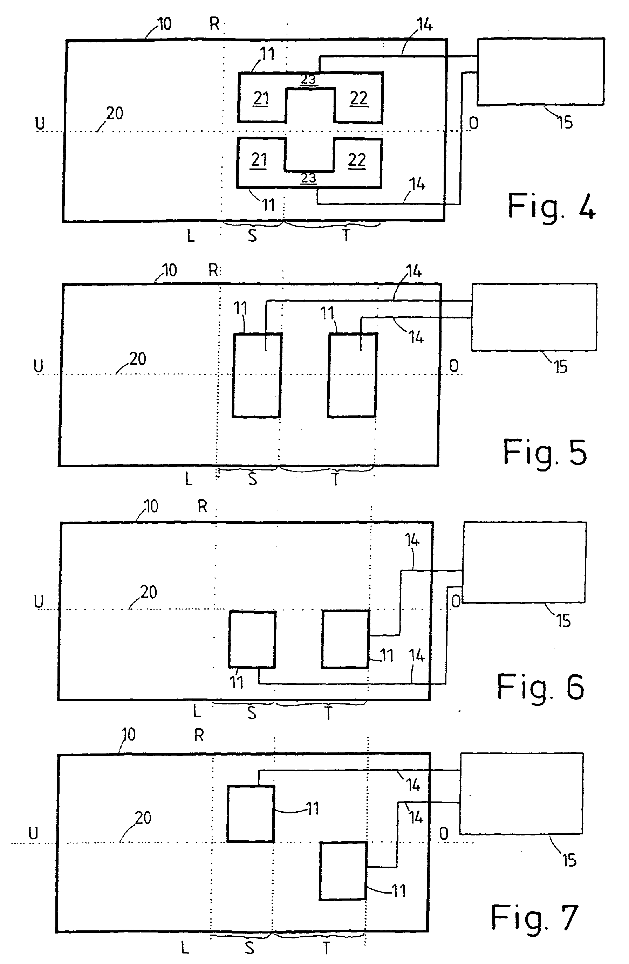

Fig. 4 zeigt eine bevorzugte Ausführungsform der Matratze 10 mit zwei Sensorkissen 11, die über Messleitungen 14 mit der Auswerteeinheit 15 in Verbindung stehen. Die in Fig. 4 dargestellte Anordnung entspricht der Anordnung aus Fig. 1 in einer Ansicht der Matratze 10 von oben. Die beiden Sensorkissen 11 befinden sich jeweils auf einer Seite der Matratzenlängsachse 20, d.h. ein Sensorkissen befindet sich auf der linken Matratzenseite L während sich das andere Sensorkissen 11 auf der rechten Matratzenseite R befindet.

Die in Fig. 4 dargestellten Sensorkissen 11 weisen jeweils eine U-förmige Gestalt auf. Sie bestehen jeweils aus drei Kammern, nämlich aus zwei äußeren Kammern 21, 22, sowie eine die äußeren Kammern 21, 22 verbindende innere Kammer 23.

Die Sensorkissen 11 in Fig. 4 erstrecken sich ferner jeweils sowohl über den Sakral- als auch Thoraxbereich S bzw. T der Matratze, also über die Bereiche, wo der Unterleib bzw. der Brustbereich einer auf der Matratze liegenden Person zu Ruhen kommt. Diese Anordnung eignet sich insbesondere zur Aufnahme von Bewegungsdaten der Person, sowie Daten über die Richtung der Bewegung, nämlich ob sich die Person zur rechten oder linken Matratzenseite R bzw. L hin bewegt bzw. dreht. Ferner eignet sich diese Anordnung der Sensorkissen auch besonders gut zur Aufnahme von Vitalparametern, da insbesondere der Thoraxbereich T großflächig abgedeckt ist.

Mit der in Fig. 5 gezeigten Anordnung ist eine Bewegungsrichtung zum Kopf- bzw. Fußende der Matratze 10, d.h. zum oberen Matratzenende 0 bzw. unteren Matratzenende U, hin aufnehmbar, da zwei Sensorkissen an verschiedenen Positionen entlang der Matratzenlängsachse 20 angeordnet sind. Eine Aufnahme der Bewegungsrichtung zur rechten bzw. linken Matratzenseite R bzw. L hin ist jedoch nicht möglich. Im übrigen entspricht diese Anordnung der Anordnung gemäß Fig. 4, insbesondere im Hinblick auf die Messleitungen 14 und die Auswerteeinheit 15.

Fig. 6 zeigt ein weiteres Ausführungsbeispiel einer Matratze 10 mit zwei Sensorkissen 11, wobei beide Sensorkissen 11 auf einer Seite der Matratzenlängsachse 20 und zwar auf der gleichen Seite angeordnet sind. Je ein Sensorkissen 11 befindet sich im Sakralbereich S und im Thoraxbereich T. Im übrigen entspricht die Anordnung den Fig. 4 bis 5, insbesondere was die Messleitungen 14 und die Auswerteeinheit 15 betrifft. Auch mit dieser Anordnung lässt sich die Bewegung, insbesondere eine Bewegung nach rechts oder links sowie zum Kopf- bzw. Fußende der Matratze hin aufnehmen, da die Sensorkissen 11 sich jeweils nicht symmetrisch zur Matratzenlängsachse 20 ausdehnen, sondern vielmehr einseitig an der Matratze angeordnet sind.

Fig. 7 zeigt ein weiteres Ausführungsbeispiel mit zwei Sensorkissen, von denen eins im Sakralbereich S auf der rechten Matratzenseite R und das andere Sensorkissen 11 auf der linken Matratzenseite L im Thoraxbereich T angeordnet ist. Auch mit dieser Anordnung lassen sich Bewegungen zur rechten Matratzenseite R oder zur linken Matratzenseite L sowie zum oberen Matratzenende 0 bzw. zum unteren Matratzenende U hin aufnehmen. Ferner können Vitalparameter gemessen werden, insbesondere mit dem im Thoraxbereich T angeordneten Sensorkissen 11. Im übrigen entspricht die Vorrichtung gemäß Fig. 7 den Fig. 4 bis 6, insbesondere was die Messleitungen 14 sowie die Auswerteeinheit 15 anbelangt.

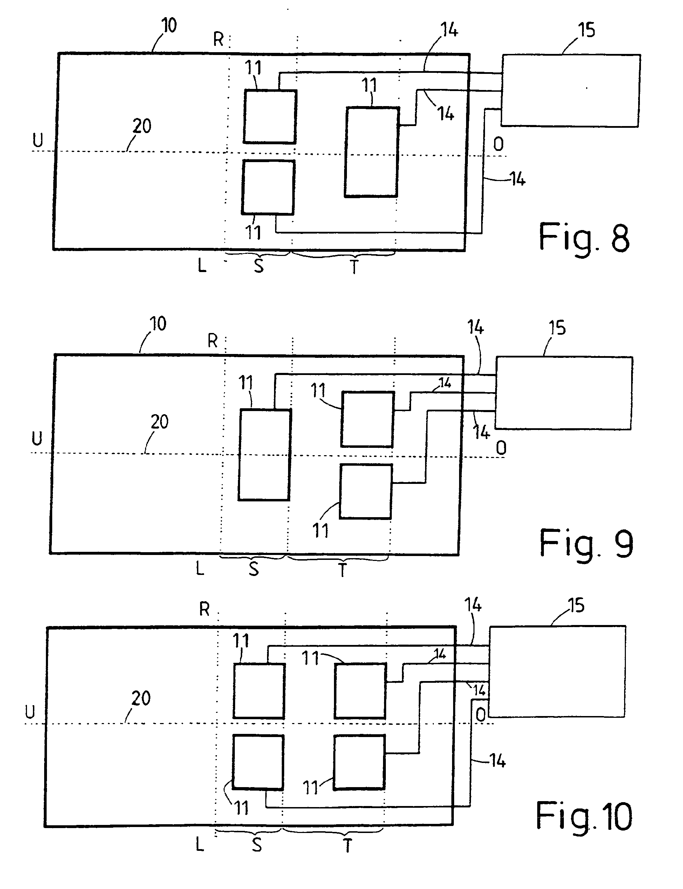

Fig. 8 zeigt ein Ausführungsbeispiel einer Matratze 10 mit drei Sensorkissen 11. Zwei der Sensorkissen 11 befinden sich im Sakralbereich S, während sich das dritte Sensorkissen im Thoraxbereich T befindet. Die beiden im Sakralbereich angeordneten Sensorkissen sind derart angeordnet, dass sich je ein Sensorkissen auf einer Seite der Matratzenlängsachse 20 befindet, wobei ein Sensorkissen 11 sich auf der linken Matratzenseite L und das anderen auf der rechten Matratzenseite R befindet. Das sich im Thorax befindende Sensorkissen 11 erstreckt sich hingegen sowohl über die linke Matratzenhälfte als auch über die rechte Matratzenhälfte. Das sowohl die rechte, als auch die linke Matratzenhälfte bedeckende Sensorkissen 11 im Thoraxbereich T ist größer ausgebildet als jeweils die sich nur auf einer Matratzenseite befindenden Sensorkissen im Sakralbereich S. Durch diese Anordnung lässt sich ebenfalls eine Bewegung der auf der Matratze 10 liegenden Person, sowie deren Richtung zur rechten bzw. linken Matratzenseite R bzw. L sowie zum oberen Matratzenende 0 und zum unteren Matratzenende U hin aufnehmen. Im übrigen entspricht die Anordnung den Anordnungen gemäß den Fig. 4 bis 7, insbesondere was die Messleitungen 14 und die Auswerteeinheit 15 betrifft.

Fig. 9 zeigt eine weitere Anordnung mit drei Sensorkissen 11, die ähnlich zu der in der Fig. 8 gezeigten Anordnung ist. Jedoch sind die sich im Thoraxbereich T und im Sakralbereich S befindenden Sensorkissen 11 vertauscht, so dass sich im Sakralbereich S lediglich ein Sensorkissen 11 symmetrisch und rechts und links zur Matratzenlängsachse 20 befindet. Im Thoraxbereich T befinden sich hingegen auf jeder Matratzenseite R bzw. L jeweils nur ein Sensorkissen 11. Im übrigen entspricht die Anordnung den in den Fig. 4 bis 8 gezeigten Anordnungen, insbesondere was die Messleitungen 14 und die Auswerteeinheit 15 betrifft.

Fig. 10 zeigt ein Ausführungsbeispiel mit vier Sensorkissen 11. Bei dieser Anordnung sind jeweils zwei Kissen im Sakralbereich S und im Thoraxbereich T angeordnet. Sowohl im Sakralbereich S als auch im Thoraxbereich T befindet sich jedoch jeweils nur ein Sensorkissen 11 auf jeder Matratzenseite R bzw. L. Diese Anordnung hat den Vorteil, dass sie neben der Bewegung der Person, sowie deren Richtung zur rechten bzw. linken Matratzenseite R bzw. L bzw. zum oberen Matratzenende 0 bzw. zum unteren Matratzenende U auch die genaue Schwerpunktlage der Person ermitteln kann. Grundsätzlich kann zwar auch mit drei Sensorkissen gemäß den Anordnungen der Fig. 8 und 9 ein Schwerpunkt ermittelt werden. Mit vier Sensorkissen 11 ist jedoch die Ermittlung der Schwerpunktlage wesentlich genauer.

Im übrigen können mit der Vorrichtung gemäß Fig. 10 auch die Vitalparameter, wie oben beschrieben, ermittelt werden. Die Vorrichtung nach Fig. 10 entspricht im übrigen den Fig. 4 bis 9, insbesondere was die Messleitungen 14 und die Auswerteeinheit 15 betrifft.

Die erfindungsgemäßen Sensorkissen erlauben den Messbereich eines Drucksensors 13 wesentlich zu vergrößern, indem an den Druckaufnehmer 12 ein mit einem druckübertragenden Medium gefülltes Kissen 11 verbunden ist. Hierdurch wird jedoch nicht nur die Messfläche vergrößert. Vielmehr wird auch der Messbereich eines Druckaufnehmers erhöht, da der Druckaufnehmer nunmehr nicht nur kleine Drücke, sondern auch sehr große Drücke aufnehmen kann. Dies ist bei einem isoliert angeordneten Druckaufnehmer ohne verbundenem Sensorkissen nicht möglich.

Durch die Vergrößerung der Messfläche gestattet der erfindungsgemäße Drucksensor den Aufbau einer erfindungsgemäßen Matratze mit nur sehr wenigen Drucksensoren, insbesondere mit nur zwei, drei oder vier Drucksensoren, trotz Abdeckung einer großen Messfläche. Ein komplizierter, aufwendiger und störungsanfälliger Aufbau mit einer Vielzahl kleiner einzelner Druckaufnehmer kann somit vermieden werden.

Dank der Erfindung kann eine Matratze mit relativ großflächigen Sensoren ausgestattet werden, so dass mit einer möglichst geringen Anzahl von Sensoren und einer möglichst geringen Beeinträchtigung der Liegequalität und des Bettklimas die für einen großen Teil der Anwendungen in der Pflege oder der medizinischen Anwendungen ausreichenden Parameter der Bewegung und der sonstigen Vitalparameter erfasst werden können. Hierzu sieht die Erfindung vor, Sensorgrößen, -gestaltung und -anordnung so zu wählen, dass mit nur zwei bis fünf Sensoren eine ausreichende Erkennung und Qualifizierung von Bewegungen sowie eine Messung der über Druck und Bewegungssensoren erfassbaren Vitalparameter, nämlich Herzrate, Atmung und eventueller Muskeltremor, möglich ist. Alternativ oder zusätzlich zu den beschriebenen Sensorkissen kann man auch großflächige Halbleitersensoren verwenden.

Die Erfindung ermöglicht insgesamt jede Bewegung einer sich auf einem Bett befindenden Person zu ermitteln, wobei man auch erkennen kann, wie stark die Bewegung ist. Die vorgeschlagenen Sensoren erlauben auch zu erkennen, ob eine Bewegung eine relevante Lageänderung der Person, bspw. ein auf die Seite Drehen oder Umdrehen, bedeutet oder es sich nur um ein "Zucken" handelt. Hierzu erfolgt insbesondere eine kontinuierliche Messung der Messwerte, insbesondere der Änderung der Form bzw. eine Verformung der Sensoren. Hierbei kann bspw. ein Druck innerhalb eines Sensorkissens gemessen werden oder bspw. ein anderes Messsignal, wie es bspw. bei piezoelektrischen oder anderen Elementen abgegriffen werden kann.

Die wenigen Sensoren sind großflächig ausgebildet und in einer gezielten räumlichen Anordnung in oder an der Matratze oder deren Unterfederung angeordnet. Insbesondere handelt es sich um eine asymmetrische Anordnung der Sensoren, wodurch nicht nur ermittelt werden kann, wo die Person im Bett liegt, sondern auch wie die Person liegt, d.h. auf dem Rücken, auf der Seite oder auf dem Bauch o.ä. Es ist jedoch auch eine symmetrische Anordnung der Sensoren möglich.

Durch die Anordnung wenigstens eines Sensors im Thoraxbereich lassen sich dynamische Werte der Person messen, insbesondere Herzschlag und Atmung. Durch die kontinuierliche Messung und insbesondere eine ständige Aufzeichnung der Messwerte entsteht ein Protokoll über das Liegeverhalten der Person. Hierdurch lässt sich kontrollieren, ob eine pflegebedürftige Person oder ein Patient von einem Pfleger regelmäßig umgelagert worden ist oder nicht. Dies ist insbesondere im Hinblick auf Qualitätskontrollen in Pflegeheimen und Krankenhäusern vorteilhaft, da aufgrund in diesem Bereich herrschenden Personalmangels Pflegemaßnahmen immer häufiger nicht mit der gewünschten Qualität durchgeführt werden.

Zusätzlich oder alternativ zu den erläuterten Sensorkissen sind Sensoren an oder in der die Matratze tragenden Unterfederung, insbesondere an oder in federnden Auflagetellern einer Unterlage für die Matratze angeordnet. Diese Sensoren weisen bspw. Dehnungsmessstreifen auf. Von der Matratze werden dann die Bewegungen der sich auf der Matratze befindenden Person großflächig auf die sich vorzugsweise punktuell unter der Matratze befindenden Auflageteller der Unterfederung übertragen. Die sich einstellende Verformung der Unterteller bzw. Unterfederung überträgt sich auf die angebrachten Dehnungsmessstreifen, die eine dieser Verformung proportionale Messwertänderung liefern.

Insgesamt erreicht man dank der besonderen Anordnung der Sensoren an nur ausgewählten Bereichen an einer Matratze bzw. deren Unterfederung, die besonderen Bereichen des Körpers einer sich auf der Matratze befindenden Person zugeordnet sind, insbesondere im Bereich des Thorax bzw. im Sakralbereich dieser Person, eine Messwerterfassung, die sehr aussagekräftige Daten über das Liegeverhalten der Person und damit über die Durchführung von Pflegemaßnahmen erlaubt.

### Bezugszeichenliste:

- 10: Matratze
- 11: Sensorkissen
- 12: Sensorelement/ Druckaufnehmer
- 13: Drucksensor
- 14: Messleitung
- 15: Auswerteeinheit
- 16: Steuerungseinheit
- 17: Datenleitung
- 18: Steuerungsleitung
- 19: Schlauch/Schlauchkapillare
- 20: Matratzenlängsachse
- 21: äußere Kammer
- 22: äußere Kammer
- 23: innere Kammer

- S: Sakralbereich
- T: Thoraxbereich
- R: rechte Matratzenseite
- L: linke Matratzenseite
- O: oberes Matratzenende
- U: unteres Matratzenende

## Patentansprüche

1. Vorrichtung zum Messen von Werten einer auf einer Matratze (10) liegenden Person, insbesondere deren Bewegung, Bewegungsrichtung und/oder Vitalparameter, mit bis zu fünf der Matratze (10) zugeordneten und jeweils einen großflächigen Messbereich abdeckenden Sensoren, **dadurch gekennzeichnet, dass** die Sensoren im Thorax- (T) und Sakralbereich (S) an und/oder in der Matratze angeordnet sind, wobei die Sensoren im Thoraxbereich (T) zum Messen dynamischer Werte, wie Herzschlag und/oder Atmung, und die Sensoren im Thorax- (T) und/oder Sakralbereich (S) zum Messen im Wesentlichen statischer Werte wie zur Erfassung einer Rücken-, Seiten-, oder Bauchlage der Person ausgebildet sind.

2. Vorrichtung nach Anspruch 1, **dadurch gekennzeichnet, dass** sie eine Unterfederung zur Aufnahme der Matratze (10) aufweist, an der Sensoren angebracht sind, insbesondere an Auflagetellern der Unterfederung, wobei wenigstens ein Sensor einen Dehnungsmessstreifen aufweist und wenigstens ein Sensor derart ausgebildet ist, dass eine Änderung seiner Form messbar ist.

3. Vorrichtung nach einem der vorherigen Ansprüche, **dadurch gekennzeichnet, dass** eine kontinuierliche Messung und Aufzeichnung der Messwerte der Sensoren im Thorax- und/oder Sakralbereich erfolgt.

4. Vorrichtung nach einem der vorherigen Ansprüche, **dadurch gekennzeichnet, dass** wenigstens ein Sensor als Drucksensor (13) ausgebildet ist, der ein Sensorkissen (11) aufweist, das mit einem gasförmigen oder flüssigen Medium gefüllt ist, wobei ein dem Sensorkissen (11) von außen zugeführter Druck über das Medium auf ein Sensorelement (12) übertragbar ist und das Sensorkissen (11) im Bereich der Liegefläche in oder an der Matratze (10) angeordnet ist.

5. Vorrichtung nach Anspruch 1, **dadurch gekennzeichnet, dass** zwei Sensorkissen (11) vorgesehen sind, wobei
a) ein erstes im Sakralbereich (S) und ein zweites im Thoraxbereich (T) angeordnet ist und beide Sensorkissen (11) im wesentlichen symmetrisch zur Matratzenlängsachse (20) angeordnet sind,
b) ein erstes im Sakralbereich (S) und ein zweites im Thoraxbereich (T) angeordnet ist und beide Sensorkissen (11) im wesentlichen rechts oder links der Matratzenlängsachse (20) angeordnet sind,
c) ein erstes im Sakralbereich (S) und ein zweites im Thoraxbereich (T) angeordnet ist, von denen ein Sensorkissen (11) rechts und das andere Sensorkissen (11) links der Matratzenlängsachse (20) angeordnet ist, oder
d) jedes sowohl im Sakralbereich (S) als auch im Thoraxbereich (T) angeordnet ist und beide Sensorkissen (11) im wesentlichen symmetrisch zur Matratzenlängsachse (20) angeordnet sind.

6. Vorrichtung nach einem der vorherigen Ansprüche, **dadurch gekennzeichnet, dass** drei Sensorkissen (11) vorgesehen sind, von denen
a) zwei im Thoraxbereich (T) und das dritte im Sakralbereich (S) angeordnet ist, oder
b) zwei im Sakralbereich (S) und eins im Thoraxbereich (T) angeordnet ist.

7. Vorrichtung nach Anspruch 1, **dadurch gekennzeichnet, dass** vier Sensorkissen (11) vorgesehen sind, von denen je zwei im Sakralbereich (S) und im Thoraxbereich (T), insbesondere symmetrisch zur und außerhalb der Matratzenlängsachse (20), angeordnet sind.

8. Vorrichtung nach Anspruch 6, **dadurch gekennzeichnet, dass** das Sensorkissen (11) zusätzlich mit einem elastischen Material, insbesondere Schaumstoff, gefüllt ist und/ oder über einen Schlauch bzw. eine Schlauchkapillare (19) mit dem Sensorelement (12) verbunden ist und jedes Sensorelement (12) ein Druckaufnehmer ist.

9. Vorrichtung nach einem der vorherigen Ansprüche, **dadurch gekennzeichnet, dass** wenigstens ein, insbesondere zwei oder alle, Sensorkissen (11) U-förmig ausgebildet ist und drei Kammern (21, 22, 23) aufweist, nämlich zwei äußere Kammern (21, 22) und eine innere Kammer (23), die äußeren Kammern (21, 22) verbindende Kammer, wobei insbesondere die innere Kammer (23) kleiner ist als jede der äußeren Kammern (21, 22).

10. Vorrichtung nach einem der vorherigen Ansprüche, **dadurch gekennzeichnet, dass** sie eine Auswerteeinheit (15) zum Auswerten der gemessenen Werte aufweist, wobei die Sensoren bzw. Sensorelemente (12) mit der Auswerteeinheit (15) verbunden sind, und die Matratze (10) zusätzlich mit der Auswerteeinheit (15) verbundene Temperatursensoren aufweist.

11. Vorrichtung nach einem oder mehreren der Ansprüche 4 bis 10, **dadurch gekennzeichnet, dass** mit den Sensorkissen (11) gemessene Drücke in der Auswerteeinheit (15) auswertbar sind, indem eine Verrechnung der Absolutdrücke der Sensorkissen (11) und/oder Relativdrücke zwischen mehreren Sensorkissen (11) erfolgt, um eine Bewegung, deren Richtung und/oder Vitalparameter der Person zu ermitteln.

12. Vorrichtung nach einem der vorherigen Ansprüche, **dadurch gekennzeichnet, dass** auch eine Messung von Temperaturwerten in der Matratze (10) erfolgt, insbesondere im Bereich der Sensorkissen (11), und diese Temperaturwerte mit den gemessenen Drücken verrechenbar sind, wobei auch der zeitliche Verlauf der Drücke und/oder Temperaturwerte verrechenbar ist.

13. Vorrichtung nach einem der vorherigen Ansprüche, **dadurch gekennzeichnet, dass** nach dem Auswerten der Drücke und ggf. Temperaturwerte die Matratze (10) ganz oder teilweise, insbesondere ein Matratzenkern, in Bewegung versetzbar ist, um die Person zu bewegen bzw. zu stimulieren.

## Claims

1. A device for measuring values from a person lying on a mattress (10), in particular the movement, direction of movement and/or vital parameters of said person, using up to five sensors assigned to the mattress, with each sensor covering a large measuring area, **characterized in that** the sensors are arranged on and/or in the mattress (10) in the region of the thorax (T) and/or the sacrum (S), with the sensors in the region of the thorax (T) being designed for measuring dynamic values such as heartbeat and/or respiration, and the sensors in the region of the thorax (T) and/or the sacrum (S) being designed for measuring substantially static values such as those employed for determining whether the person is lying on his or her back, side or stomach.

2. The device according to Claim 1, **characterized in that** it has a sprung support for holding the mattress (10), on which the sensors are arranged, in particular on bearing plates of the sprung support, with at least one sensor having a wire strain gauge and at least one sensor being designed in such a manner that it is possible to measure a change in its shape.

3. The device according to one of the previous Claims, **characterized in that** the measurement values of the sensors in the region of the thorax and/or the sacrum are measured and recorded continuously.

4. The device according to one of the previous Claims, **characterized in that** at least one sensor is designed as a pressure sensor (13) which has a sensor cushion (11) filled with a gaseous or liquid medium, it being possible for pressure that is exerted on the sensor cushion (11) from the outside to be transmitted via the medium to a sensor element (12), and the sensor cushion (11) being arranged in or on the mattress (10) in the region of the lying surface.

5. The device according to Claim 1, **characterized in that** two sensor cushions (11) are provided, wherein
a) a first is arranged in the region of the sacrum (S) and a second is arranged in the region of the thorax (T), and both sensor cushions (11) are arranged substantially symmetrically with respect to the longitudinal axis (20) of the mattress,
b) a first is arranged in the region of the sacrum (S) and a second is arranged in the region of the thorax (T), and both sensor cushions (11) are arranged substantially to the right or left of the longitudinal axis (20) of the mattress,
c) a first is arranged in the region of the sacrum (S) and a second is arranged in the region of the thorax (T), and of these one sensor cushion (11) is arranged to the right and the other sensor cushion (11) is arranged to the left of the longitudinal axis (20) of the mattress, or
d) each is arranged both in the region of the sacrum (S) and in the region of the thorax (T), and both sensor cushions (11) are arranged substantially symmetrically with respect to the longitudinal axis (20) of the mattress.

6. The device according to one of the previous Claims, **characterized in that** three sensor cushions (3) are provided, of which
a) two are arranged in the region of the thorax (T) and the third is arranged in the region of the sacrum (S), or
b) two are arranged in the region of the sacrum (S) and one is arranged in the region of the thorax (T).

7. The device according to Claim 1, **characterized in that** four sensor cushions (11) are provided, of which two are arranged in the region of the sacrum (S) and two are arranged in the region of the thorax (T), in particular symmetrically with respect to, and outside of, the longitudinal axis (20) of the mattress.

8. The device according to Claim 6, **characterized in that** the sensor cushion (11) is additionally filled with an elastic material, in particular foam, and/or is connected to the sensor element (12) via a tube or flexible capillary (19), and that each sensor element (12) is a pressure pick-up.

9. The device according to one of the previous Claims, **characterized in that** at least one sensor cushion (11), in particular two or all the sensor cushions (11), is/are of U-shaped design and has/have three chambers (21, 22, 23), namely two outer chambers (21, 22) and one inner chamber (23) connecting the two outer chambers (21, 22), in particular with the inner chamber (23) being smaller than any of the outer chambers (21, 22).

10. The device according to one of the previous Claims, **characterized in that** it has an evaluation unit (15) for evaluating the measured values, the sensors or sensor elements (12) being connected to the evaluation unit (15), and the mattress (10) having in addition temperature sensors connected to the evaluation unit (15).

11. The device according to one or more of Claims 4 to 10, **characterized in that** pressures measured with the sensor cushions (11) can be evaluated in the evaluation unit (15) **in that** absolute pressures from the sensor cushions and/or relative pressures between a plurality of sensor cushions (11) are combined in a calculation to determine a movement, the direction of said movement and/or vital parameters of the person.

12. The device according to one of the previous Claims, **characterized in that** temperature values are also measured in the mattress (10), in particular in the region of the sensor cushions (11), and that these temperature values can be included in the calculation with the measured pressures, it also being possible to calculate the chronological progression of the pressures and/or temperature values.

13. The device according to one of the previous Claims, **characterized in that**, following evaluation of the pressures and, if appropriate, temperature values, the mattress (10) in its entirety or in part, in particular a mattress core, can be set in motion in order to move or stimulate the person.

## Revendications

1. Dispositif de mesure de valeurs d'une personne allongée sur un matelas (10), notamment son mouvement, le sens du mouvement et/ou des paramètres vitaux, comprenant jusqu'à cinq capteurs affectés au matelas (10) et couvrant respectivement une zone de mesure étendue, **caractérisé en ce que** les capteurs sont disposés dans la zone du thorax (T) et sacrale (S) sur et/ou dans le matelas, les capteurs dans la zone du thorax (T) étant conçus pour mesurer des valeurs dynamiques comme les battements du coeur et/ou la respiration et les capteurs dans la zone du thorax (T) et/ou sacrale (S) pour mesurer des valeurs essentiellement statiques ainsi que pour détecter une position sur le dos, sur le côté ou sur le ventre de la personne.

2. Dispositif selon la revendication 1, **caractérisé en ce qu'**il présente un sommier pour recevoir le matelas (10), sur lequel sont montés des capteurs, notamment au niveau des coupelles d'appui du sommier, au moins un capteur présentant une jauge de contrainte et au moins un capteur étant configuré de telle sorte qu'il est possible de mesurer une modification de sa forme.

3. Dispositif selon l'une des revendications précédentes, **caractérisé en ce qu'**une mesure et un enregistrement continus des valeurs mesurées des capteurs se déroulent dans la zone du thorax et/ou sacrale.

4. Dispositif selon l'une des revendications précédentes, **caractérisé en ce que** l'au moins un capteur est réalisé sous la forme d'un capteur de pression (13) qui présente un coussin de détection (11) rempli d'un fluide gazeux ou liquide, la pression exercée depuis l'extérieur sur le coussin de détection (11) pouvant être transmise par le biais du fluide à un élément de détection (12) et le coussin de détection (11) étant disposé dans la zone de la surface de couchage sur ou dans le matelas (10).

5. Dispositif selon la revendication 1, **caractérisé en ce que** deux coussins de détection (11) sont prévus,
a) un premier étant disposé dans la zone sacrale (S) et un deuxième dans la zone du thorax (T) et les deux coussins de détection (11) étant disposés pour l'essentiel de manière symétrique par rapport à l'axe longitudinal du matelas (20),
b) un premier étant disposé dans la zone sacrale (S) et un deuxième dans la zone du thorax (T) et les deux coussins de détection (11) étant disposés pour l'essentiel à droite ou à gauche de l'axe longitudinal du matelas (20),
c) un premier étant disposé dans la zone sacrale (S) et un deuxième dans la zone du thorax (T), parmi ceux-ci un coussin de détection (11) est disposé à droite et l'autre coussin de détection est disposé à gauche de l'axe longitudinal du matelas (20), ou
d) chacun étant disposé à la fois dans la zone sacrale (S) et dans la zone du thorax (T) et les deux coussins de détection (11) étant disposés pour l'essentiel de manière symétrique par rapport à l'axe longitudinal du matelas (20).

6. Dispositif selon l'une des revendications précédentes, **caractérisé en ce que** trois coussins de détection (11) sont prévus, parmi lesquels
a) deux sont disposés dans la zone du thorax (T) et le troisième dans la zone sacrale (S) ou
b) deux sont disposés dans la zone sacrale (S) et le troisième dans la zone du thorax (T).

7. Dispositif selon la revendication 1, **caractérisé en ce que** quatre coussins de détection (11) sont prévus, parmi lesquels deux sont à chaque fois disposés dans la zone sacrale (S) et dans la zone du thorax (T), notamment de manière symétrique par rapport à l'axe longitudinal du matelas (20).

8. Dispositif selon la revendication 6, **caractérisé en ce que** le coussin de détection (11) est en plus rempli d'un matériau élastique, notamment de la mousse et/ou est relié par le biais d'un tuyau ou d'un capillaire flexible (19) avec l'élément de détection (12) et chaque élément de détection (12) est un capteur de pression.

9. Dispositif selon l'une des revendications précédentes, **caractérisé en ce qu'**au moins un, notamment deux ou tous les coussins de détection (11) sont réalisés en forme de U et présentent trois chambres (21, 22, 23), à savoir deux chambres extérieures (21, 22) et une chambre intérieure (23) qui relie les chambres extérieures (21, 22), la chambre intérieure (23) étant notamment plus petite que chacune des chambres extérieures (21, 22).

10. Dispositif selon l'une des revendications précédentes, **caractérisé en ce qu'**il présente une unité d'interprétation (15) pour interpréter les valeurs mesurées, les capteurs ou les éléments de détection (12) étant reliés avec l'unité d'interprétation (15) et le matelas (10) présentant en plus des capteurs de température reliés avec l'unité d'interprétation (15).

11. Dispositif selon une ou plusieurs des revendications 4 à 10, **caractérisé en ce que** les pressions mesurées avec les coussins de détection (11) peuvent être interprétées dans l'unité d'interprétation (15) en effectuant un calcul des pressions absolues des coussins de détection (11) et/ou des pressions relatives entre plusieurs coussins de détection (11) afin de déterminer un mouvement, sa direction et/ou les paramètres vitaux de la personne.

12. Dispositif selon l'une des revendications précédentes, **caractérisé en ce qu'**une mesure des valeurs de température a également lieu dans le matelas (10), notamment dans la zone des coussins de détection (11), et ces valeurs de température peuvent être calculées avec les pressions mesurées, les pressions et/ou les valeurs de température pouvant être calculées à partir du tracé dans le temps.

13. Dispositif selon l'une des revendications précédentes, **caractérisé en ce qu'**après avoir interprété les pressions et éventuellement les valeurs de température, le matelas (10) peut être entièrement ou partiellement mis en mouvement, notamment un coeur de matelas, afin de faire bouger ou de stimuler la personne.
